# EUROPÄISCHE PATENTANMELDUNG

(11) **EP 2 926 740 A1**
(43) Veröffentlichungstag der Anmeldung: **07.10.2015**
(21) Anmeldenummer: 15000611.2
(22) Anmeldetag: 03.03.2015
(51) Int. Cl.: A61B 17/00, A61B 17/12, A61B 19/00

(54) **Medizinische Einrichtung zur Ausbildung eines implantierbaren Rings**

(30) Priorität: 04.04.2014 AT 2522014
(71) Anmelder: A.M.I. Agency for Medical Innovations GmbH, 6800 Feldkirch (AT)
(72) Erfinder: Egle, Walter, 6842 Koblach (AT)
(74) Vertreter: Hofmann, Ralf U.

(57) **Zusammenfassung**

Eine medizinische Einrichtung (1) zur Ausbildung eines implantierbaren Rings, insbesondere zur Drainage oder zum Einschnüren oder Markieren von Gewebe, umfasst einen flexiblen Strang (2) und zumindest ein Kupplungsstück (3) zum Verbinden eines ersten Endes (4) und eines zweiten Endes (5) des flexiblen Strangs (2). Das Kupplungsstück (3) weist einen Hohlraum (6) zur Aufnahme von Klebstoff (7) auf und vom Hohlraum (6) ist durch zumindest eine Klebstoffaustrittsöffnung (8) Klebstoff (7) in einen Verbindungsbereich (9) zwischen dem ersten Ende (4) des flexiblen Strangs (2) und dem Kupplungsstück (3) zuführbar. (Fig. 6)

## Beschreibung

Die vorliegende Erfindung betrifft eine medizinische Einrichtung zur Ausbildung eines implantierbaren Rings, insbesondere zur Drainage oder zum Einschnüren oder Markieren von Gewebe, umfassend einen flexiblen Strang und zumindest ein Kupplungsstück zum Verbinden eines ersten Endes und eines zweiten Endes des flexiblen Strangs.

Zur Behandlung von Patienten werden häufig schlauchförmige, flexible Stränge (= Schläuche) eingesetzt, um vorhandene anatomische Strukturen wie z.B. die Harnleiter, den Gallengang, Gefäße, Fistelgänge und Magenein- und Magenausgang offenzuhalten oder Defekte zu überbrücken. Eine spezifische Anwendung schlauchförmiger flexibler Stränge ist das sogenannte "Drainieren" von Wundgebieten. Dabei wird ein Ende eines solchen flexiblen Strangs in das Wundgebiet gelegt, während das andere Ende den Körper verlässt und in einen Behälter zum Auffangen des durch den Hohlraum des Stranges entweichenden Wundsekretes angeschlossen ist.

Bei manchen Anwendungen in der Medizin ist es nötig, die Enden solcher flexibler Stränge intraoperativ zusammenzufügen, um einen geschlossenen Ring auszubilden. Dies ist beispielsweise bei der Anlage einer Langzeitdrainage einer rekto-analen Fistel notwendig und wird auch als Fadendrainage oder Seton bezeichnet. Die Drainage dient der Beruhigung der Entzündung der Fistel. Der flexible Strang verhindert dabei das Verstopfen der Fistel, wobei das Wundsekret an der Oberfläche des Ringes entlang zur Hautoberfläche austreten kann.

Die Fistel wird sondiert und der flexible Strang in die Fistel eingeführt. Häufig werden in der Praxis Drainagen angelegt, bei denen die beiden Enden des flexiblen Strangs außerhalb des Körpers des Patienten vom Operateur verknotet werden. Der Knoten einer derart hergestellten Drainage liegt im Bereich des Anus und bereitet den betroffenen Patienten in Folge oft Schwierigkeiten im Alltag. Es können Schmerzen beim Sitzen und häufig Hautirritationen rund um den Fistelgang auftreten.

Es sind bereits implantierbare Ringe zum Drainieren von rekto-analen Fisteln bekannt geworden, bei denen ein Kupplungsstück die beiden Enden des flexiblen Strangs verbindet. Diese Ausführung hat den Vorteil, dass ein glatter, geschlossener und flexibler Ring ohne Verdickungen und Knoten hergestellt werden kann, welcher Schmerzen und Hautirritationen beim Patienten verringert. In der Praxis wurde allerdings festgestellt, dass sich die Steckverbindungen unerwünscht lösen können, was einen neuerlichen Arzttermin zur Anlage eines neuen Rings zur Drainage zur Folge hat.

Weitere Anwendungen geschlossener implantierbarer Ringe sind beispielsweise die Magen-Bypass-Chirurgie und die Vertical-Banded-Gastroplasty zur Behandlung von Adipositas. Die medizinischen Einrichtungen werden im Körper des Patienten zu einem Ring zusammengefügt. Weiters ist es auch möglich, geschlossene Ringe zur intraoperativen Markierung von Gewebebereichen in Form einer langzeitimplantatfähigen Schlaufe einzusetzen und diese für spätere Behandlungen, insbesondere zur Orientierung bei Strahlentherapien zu verwenden. Auch das dauerhafte aber reversible Abschnüren von Hohlorganen, z.B. an den Eileitern zur temporären Sterilisation oder um eine intraoperative Bestrahlung zu ermöglichen, ist mittels solcher medizinischer Einrichtungen realisierbar.

Aufgabe der Erfindung ist es, eine zuverlässige knotenfreie Verbindung der Enden eines Strangs zur Ausbildung eines implantierbaren Rings zu realisieren.

Erfindungsgemäß gelingt dies durch eine medizinische Einrichtung zur Ausbildung eines implantierbaren Rings mit dem Merkmal des Anspruchs 1.

Bei einer medizinischen Einrichtung gemäß der Erfindung ist vorgesehen, dass das Kupplungsstück einen Hohlraum zur Aufnahme von Klebstoff aufweist und vom Hohlraum durch zumindest eine Klebstoffaustrittsöffnung Klebstoff in einen Verbindungsbereich zwischen dem ersten Ende des flexiblen Strangs und dem Kupplungsstück zuführbar ist.

Es kann dadurch in einfacher und vorteilhafter Weise intraoperativ eine Verklebung zur Verbindung des ersten Endes des flexiblen Strangs mit dem Kupplungsstück durchgeführt werden.

Im verbundenen Zustand ihrer Enden bildet die medizinische Einrichtung der Erfindung vorzugsweise einen Ring mit in Längserstreckung des flexiblen Strangs bzw. Umfangsrichtung des Rings gesehen im Wesentlichen konstantem Querschnitt.

Vorzugsweise ist das zweite Ende des flexiblen Strangs mit dem Kupplungsstück reibschlüssig und/oder formschlüssig und/oder stoffschlüssig verbunden. Diese Verbindung kann beispielsweise durch Aufschrumpfen des flexiblen Strangs auf das Kupplungsstück hergestellt sein. Auch andere Verbindungen, wie Schweißverbindungen oder Klebeverbindungen sind denkbar und möglich. Die Ausführung des mit dem zweiten Ende verbundenen Kupplungsstücks stellt auch den bevorzugten Auslieferungszustand der medizinischen Einrichtung dar, d. h. die Verbindung zwischen dem zweiten Ende des flexiblen Strangs und dem Kupplungsstück erfolgt bereits werkseitig. Die medizinische Einrichtung umfasst im Auslieferungszustand somit den flexiblen Strang und das am zweiten Ende des flexiblen Strangs angebrachte Kupplungsstück. In anderen Ausführungsformen der Erfindung ist es auch denkbar, dass das zweite Ende des flexiblen Strangs mit dem Kupplungsstück verbindbar ausgeführt ist, das heißt, dass bei diesen Ausführungen die Verbindung des zweiten Endes des flexiblen Strangs mit dem Kupplungsstück vom medizinischen Personal, vorzugsweise vor, oder aber während der Implantation, vorgenommen wird.

In einer ersten möglichen Ausführungsform der Erfindung ist vorgesehen, dass der Hohlraum des Kupplungsstücks bereits im Auslieferungszustand mit Klebstoff aufgefüllt ist und zumindest ein Verschluss zum Verhindern eines ungewollten Austritts von Klebstoff durch die zumindest eine Klebstoffaustrittsöffnung vorgesehen ist. Der Verschluss dient dem Verhindern des ungewollten Austretens von Klebstoff und auch dem Verhindern des Eintrocknens des Klebstoffs während der Lagerung der medizinischen Einrichtung. Der Verschluss kann direkt die Klebstoffaustrittsöffnung an der Oberfläche des Kupplungsstücks überdecken oder den Gang zwischen dem Hohlraum und der Klebstoffaustrittsöffnung verschließen.

Vorzugsweise ist in dieser Ausführungsform zumindest eine den Hohlraum begrenzende Wand des Kupplungsstücks durch einen auf das Kupplungsstück ausgeübten äußeren Druck in den Hohlraum eindrückbar, d. h. das Volumen des Hohlraums wird durch das Eindrücken der Wand verringert. Insbesondere wird beim Eindrücken der Wand das Material des Kupplungsstücks örtlich elastisch und/oder plastisch verformt. Es kann also von außen ein Druck auf das Kupplungsstück und über dieses auf den Klebstoff ausgeübt werden, um den Klebstoff aus der Klebstoffaustrittsöffnung zu drücken. Um dies zu ermöglichen, ist der Verschluss günstigerweise durch einen auf den Klebstoff einwirkenden und vom Klebstoff auf den Verschluss übertragenen Druck öffenbar. Wird der zum Öffnen des Verschlusses erforderliche Druck überschritten, so kommt es unter Verringerung des Volumens des Hohlraumes des Kupplungsstücks zu einem Austritt von Klebstoff aus der Klebstoffaustrittsöffnung in den Verbindungsbereich. Der Klebstoff tritt bevorzugt direkt im Verbindungsbereich aus und ermöglicht eine präzise und zuverlässige Verklebung des ersten Endes des flexiblen Strangs mit dem Kupplungsstück. Es ist denkbar und möglich, dass der Verschluss auch auf andere Art und Weise geöffnet wird, beispielsweise mittels vorheriger manueller Penetration durch das medizinische Personal, z.B. mittels einer Nadel.

Eine vorteilhafte Ausgestaltung dieser Ausführungsform der Erfindung sieht vor, dass der Verschluss von einer Membran gebildet wird. Günstigerweise ist die Membran hierbei durch einen auf den Klebstoff einwirkenden und vom Klebstoff übertragenen Druck aufreißbar und/oder durch einen auf den Klebstoff einwirkenden und vom Klebstoff übertragenen Druck vom Kupplungsstück abreißbar oder abhebbar. Die Membran könnte auch als Sollbruchstelle bezeichnet werden, welche bei Aufbringen eines entsprechenden Druckes auf- oder abreißt. Alternativ könnte auch vorgesehen sein, dass die Membran vor dem Zusammenfügen des ersten Endes des flexiblen Strangs und dem Kupplungsstück vom medizinischen Personal geöffnet oder vom Kupplungsstück abgelöst wird.

Bei der Membran kann es sich um eine separate Folie handeln, welche mit dem Kupplungsstück verbunden ist, z. B. auf der Oberfläche des Kupplungsstücks angeklebt ist. Insbesondere verdeckt die Membran hierbei Bereiche der Wand des Kupplungsstücks, welche die Klebstoffaustrittsöffnung aufweist. In anderen Ausführungsformen kann vorgesehen sein, dass sich die Membran in einem Gang zwischen dem Hohlraum und der Klebstoffaustrittsöffnung befindet.

Anstelle einer Ausbildung in Form einer separaten Membran könnte der Verschluss auch von einem Abschnitt des Kupplungsstücks selbst ausgebildet sein, welcher eine geringe Wandstärke aufweist.

In einer möglichen zweiten Ausbildungsform der Erfindung weist das Kupplungsstück zumindest eine Klebstoffeintrittsöffnung zum Einbringen von Klebstoff in den Hohlraum des Kupplungsstücks auf. Der Klebstoff kann beispielsweise mittels einer geeigneten Spritze in das Kupplungsstück injiziert werden, indem eine Kanüle oder eine entsprechende Leitung der Spritze am Kupplungsstück angesetzt oder angeschlossen wird. Es ist denkbar, dass dieser Vorgang im verbundenen Zustand des ersten Endes des flexiblen Strangs mit dem Kupplungsstück durchgeführt wird und der Klebstoff durch den auf die Spritze aufgebrachten Druck in den Verbindungsbereich verdrängt wird. Der Klebstoff wird also intraoperativ in den Hohlraum des Kupplungsstücks eingebracht. Es ist es auch denkbar und möglich, dass das Befüllen des Hohlraumes vor dem Verbinden des ersten Endes des flexiblen Strangs mit dem Kupplungsstück erfolgt und ein zusätzlicher Schritt zum Einbringen des Klebstoffs in den Verbindungsbereich durch Aufbringen eines Druckes auf eine an den Hohlraum angrenzende Wand des Kupplungsstücks erfolgt. Das Einbringen des Klebstoffs in den Hohlraum kann dann insbesondere vor dem Implantieren der Einrichtung durchgeführt werden. Auch bei diesen Ausführungen kann für die Klebstoffaustrittsöffnungen ein Verschluss vorgesehen sein. Dieser kann analog wie bei der zuvor beschriebenen ersten Ausführungsform ausgebildet sein, bei der der Hohlraum bereits im Auslieferungszustand mit Klebstoff gefüllt ist.

Sowohl in der ersten Ausführungsform, in der der Hohlraum im Auslieferungszustand befüllt ist, als auch in der zweiten Ausführungsform, in der der Hohlraum im Auslieferungszustand unbefüllt ist, befindet sich die zumindest eine Klebstoffaustrittsöffnung insbesondere in einer äußeren Oberfläche des Kupplungsstücks, z.B. in der Mantelfläche des Kupplungsstücks, am Ende eines Gangs, welcher sich vom Hohlraum zur äußeren Oberfläche des Kupplungsstücks erstreckt. Der Gang zwischen der äußeren Oberfläche des Kupplungsstücks und dem Hohlraum kann auch als Kanal oder Bohrung bezeichnet werden. Im zusammengesteckten Zustand der Enden der medizinischen Einrichtung, in welchem ein Ring ausgebildet ist, ist die Klebstoffaustrittsöffnung dann benachbart zu einer Oberfläche des flexiblen Strangs angeordnet. Ein Spalt zwischen der Klebstoffaustrittsöffnung des Kupplungsstücks und der Oberfläche des flexiblen Strangs kann in diesem Zustand mit Klebstoff gefüllt werden. Nach dem Aushärten des Klebstoffs wird eine dauerhafte Verbindung der beiden Enden des flexiblen Strangs über das Kupplungsstück hergestellt. Auch Ausbildungen, in denen der zumindest eine Gang oder zumindest einer der Gänge an einer Stirnfläche des Kupplungsstücks mündet, sind denkbar und möglich.

Der flexible Strang ist vorzugsweise schlauchförmig mit einem in Längserstreckung durchgehenden Hohlraum ausgebildet.

Zur Erleichterung des Implantierens der erfindungsgemäßen Einrichtung kann das erste Ende des flexiblen Strangs oder stattdessen das Kupplungsstück mit einer Einführsonde verbindbar sein. Eine solche Einführsonde ist vorzugsweise plastisch verformbar und weist eine längserstreckte Form auf. Vorteilhafterweise kann eine Einführsonde bereits im Auslieferungszustand der Einrichtung am ersten Ende des flexiblen Strangs oder am Kupplungsstück angebracht sein.

Weitere Merkmale und Einzelheiten bevorzugter Ausgestaltungsformen der Erfindung werden anhand der Zeichnungen erläutert. Es zeigen:
Fig. 1 eine Ansicht einer erfindungsgemäßen medizinischen Einrichtung im Auslieferungszustand gemäß einem ersten Ausführungsbeispiel der Erfindung ;
Fig. 2 und 3 die Endabschnitte der medizinischen Einrichtung gemäß Fig. 1 vor dem Zusammenfügen;
Fig. 4 einen Schnitt entlang der Schnittlinie A-A in Fig. 3;
Fig. 5 die Endabschnitte im verbundenen Zustand;
Fig. 6 und 7 einen Schnitt entlang der Schnittlinie B-B in Fig. 5 vor und nach dem Einbringen des Klebstoffs;
Fig. 8 eine Gesamtansicht der medizinische Einrichtung im verbundenen Zustand der Enden;
Fig. 9 eine schematische Darstellung einer Anwendung der erfindungsgemäßen medizinischen Einrichtung zur Drainage von rekto-analen Fisteln;
Fig. 10 eine Einführsonde;
Fig. 11 bis 16 ein zweites Ausführungsbeispiel mit zu den Fig. 2 bis 7 des ersten Ausführungsbeispiels analogen Darstellungen;
Fig. 17 bis 22 ein weiteres Ausführungsbeispiel mit zu den Fig. 2 bis 7 des ersten Ausführungsbeispiels analogen Darstellungen.

Die in den Figuren gezeigten erfindungsgemäßen Anordnungen weisen unterschiedliche Maßstäbe auf und dienen insbesondere der Veranschaulichung der Erfindung.

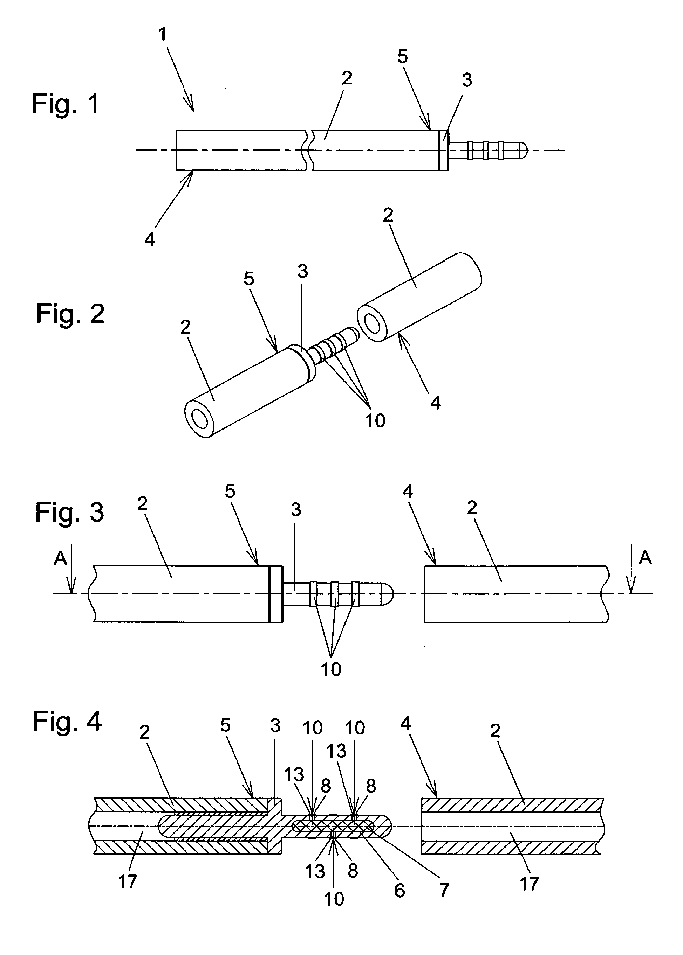

Die Fig. 1 bis 7 zeigen eine erste Ausführungsform der medizinische Einrichtung 1 zur Ausbildung eines implantierbaren Rings im Auslieferungszustand. Derartige Ringe werden insbesondere zur Drainage von rekto-analen Fisteln, zum Einschnüren von Hohlorganen oder Markieren von Gewebe verwendet. Auf die beispielhafte Anwendung zur Drainage von rekto-analen Fisteln wird weiter unten eingegangen.

Die medizinische Einrichtung 1 weist einen flexiblen Strang 2 und ein Kupplungsstück 3 auf, welches im verbundenen Zustand der medizinischen Einrichtung 1 das erste Ende 4 mit dem zweiten Ende 5 des flexiblen Strangs 2 verbindet. Die Ausbildung des Rings, d. h. das Verbinden der beiden Enden 4, 5 des flexiblen Strangs 2 über das Kupplungsstück 3 geschieht während der Implantation der medizinischen Einrichtung 1 am oder im Körper des Patienten.

Das zweite Ende 5 des flexiblen Strangs 2 ist im Auslieferungszustand der Einrichtung vorzugsweise mit dem Kupplungsstück 3 bereits verbunden. Im gezeigten Ausführungsbeispiel ist diese Verbindung stoffschlüssig in Form einer Verklebung. In den Fig. 4, 6 und 7 ist der das zweite Ende 5 mit dem Kupplungsstück 3 verbindende Klebstoff 25 dargestellt. Dies ist nicht zwingend so, vielmehr könnte das zweite Ende 5 des flexiblen Strangs mit dem Kupplungsstück 3 auch reibschlüssig und/oder formschlüssig verbunden sein. Beispielsweise ist ein Aufschrumpfen des flexiblen Strangs 2 auf das Kupplungsstück 3 ebenfalls möglich.

Die Verbindung des Kupplungsstücks 3 mit dem zweiten Ende 5 ist derart gestaltet, dass ein Fortsatz des Kupplungsstücks 3 in einen Hohlraum 17 des flexiblen Strangs 2 ragt. Auch die umgekehrte Ausbildung, bei der ein Abschnitt des flexiblen Strangs 2 am zweiten Ende 5 in eine Öffnung am Kupplungsstück 3 ragt, ist denkbar und möglich.

Das Kupplungsstück 3 weist zur Aufnahme von Klebstoff 7 einen Hohlraum 6 auf, vgl. Fig. 4, 6 und 7. Im ersten Ausführungsbeispiel ist der Hohlraum 6 des Kupplungsstücks 3 bereits im Auslieferungszustand der medizinischen Einrichtung 1 mit Klebstoff 7 aufgefüllt. Im Bereich des Kupplungsstücks 3, der zur Verbindung mit dem ersten Ende 4 des flexiblen Strangs 2 dient, weist das Kupplungsstück 3 mindestens eine Klebstoffaustrittsöffnung 8 auf. Vorteilhafterweise sind wie dargestellt mehrere in axialer Richtung (= in Richtung der Längserstreckung des flexiblen Strangs 2 im mit dem Kupplungsstück 3 verbundenen Abschnitt) beabstandete Klebstoffaustrittsöffnungen 8 vorhanden. Außerdem ist mindestens ein Verschluss 10 vorgesehen. Im Auslieferungszustand sind alle Klebstoffaustrittsöffnungen 8 durch den mindestens einen Verschluss 10 verschlossen. Im Ausführungsbeispiel ist für jede Klebstoffaustrittsöffnung 8 ein eigener Verschluss 10 vorgesehen.

Die Verschlüsse 10 sind im Ausführungsbeispiel jeweils als Membran ausgeführt, welche einen Ring um die Mantelfläche des Kupplungsstücks 3 bilden und die Klebstoffaustrittsöffnungen 8 überdecken. Die Verschlüsse 10 verhindern das ungewollte Austreten von Klebstoff 7 während der Lagerung der medizinischen Einrichtung 1 und während der Implantation. Sie dienen auch insbesondere der Verhinderung des Eintrocknens des Klebstoffs 7.

Es könnte auch ein Verschluss 10, beispielsweise in Form einer Membran vorgesehen sein, der mehrere Klebstoffaustrittsöffnungen 8 verschließt. Eine Membran zur Ausbildung des Verschlusses 10 müsste nicht unbedingt ringförmig sein. Es ist auch denkbar und möglich, dass der Verschluss 10 die Form eines Streifens, welcher wiederum nur eine der Klebstoffaustrittsöffnungen 8 oder mehrere der Klebstoffaustrittsöffnungen 8 überdeckt, aufweist. Auch könnte der Verschluss 10 eine zur Geometrie der Klebstoffaustrittsöffnung 8 ähnliche Kontur aufweisen.

Die Klebstoffaustrittsöffnungen 8 befinden sich im Ausführungsbeispiel in einer äußeren Oberfläche des Kupplungsstücks 3 am Ende jeweils eines Gangs 13, welcher den Hohlraum 6 mit der äußeren Oberfläche des Kupplungsstücks 3 verbindet.

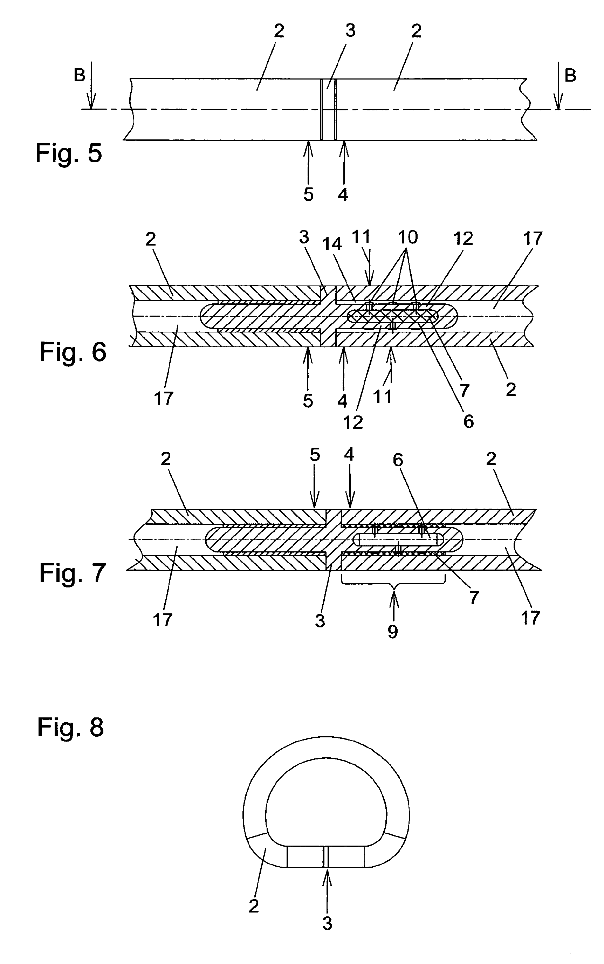

In den Fig. 2 bis 4 ist die medizinische Einrichtung 1 unmittelbar vor der Ausbildung eines zusammengefügten Zustands (Fig. 6), bei dem die Verbindung zwischen Kupplungsstück 3 und erstem Ende 4 des flexiblen Strangs 2 noch reversibel lösbar ist, gezeigt. Es sind nur die an die beiden Enden 4, 5 des Strangs 2 anschließenden Abschnitte des flexiblen Strangs 2 gezeigt. Die sich bildende gekrümmte Schleife des flexiblen Strangs 2 wurde der Übersichtlichkeit halber nicht dargestellt. Die dargestellten Abschnitte des flexiblen Strangs 2 gehören jeweils zum selben flexiblen Strang 2. Zumindest die an die Enden 4, 5 anschließenden Abschnitte des flexiblen Strangs 2 weisen einen in Längserstreckung des flexiblen Strangs 2 verlaufenden Hohlraum 17 auf, der am jeweiligen Ende 4, 5 des flexiblen Strangs 2 stirnseitig mündet. Bevorzugterweise weist der flexible Strang 2 die Form eines Schlauchs mit einem in Längsrichtung durchgehenden Hohlraum 17 auf.

Das Kupplungsstück 3 besitzt einen ersten Fortsatz, der im Hohlraum 17 des ersten Endes 4 des flexiblen Strangs 2 einsteckbar ist und einen zweiten Fortsatz, der in den Hohlraum 17 des zweiten Endes 5 ragt. Umgekehrt ist es auch denkbar und möglich, dass das Kupplungsstück 3 abgesehen vom Hohlraum 6 zumindest einen weiteren, stirnseitig mündenden Hohlraum aufweist und ein an einem der Enden 4, 5 befindlicher Fortsatz des flexiblen Strangs 2 in diesen Hohlraum ragt oder in diesen einsteckbar ist.

Sowohl im zusammengefügten (Fig. 6) als auch im dauerhaft verbundenen Zustand (Fig. 7) ist die den Hohlraum 17 begrenzende Oberfläche des flexiblen Strangs 2 den Klebstoffaustrittsöffnungen 8 benachbart. Insbesondere befindet sich zwischen der die Klebstoffaustrittsöffnungen 8 aufweisenden Oberfläche des Kupplungsstücks 3 und der den Hohlraum 17 begrenzenden Oberfläche des flexiblen Strangs 2 ein Spalt 14. Der Vorteil der Anordnung der Klebstoffaustrittsöffnungen 8 der medizinischen Einrichtung 1 unmittelbar im Verbindungsbereich 9 besteht darin, dass der Klebstoff 7 präzise in den Verbindungsbereich 9 geleitet werden kann, um die dauerhafte und zuverlässige Verbindung der beiden Enden 4, 5 des flexiblen Strangs 2 über das Kupplungsstück zu gewährleisten.

Die Fig. 6 zeigt den zusammengefügten Zustand der medizinischen Einrichtung 1, wobei der erste Fortsatz des Kupplungsstücks 3 in das erste Endes 4 des flexiblen Strangs 2 eingesteckt wurde. Um die beiden Enden 4, 5 des flexiblen Strangs 2 mit Hilfe des Kupplungsstücks 3 dauerhaft zu verbinden, wird in einem nächsten Schritt Klebstoff 7 aus dem Hohlraum 6 in den Verbindungsbereich 9 gedrückt. Der Klebstoff 7 gelangt in den Spalt 14, der sich zwischen der den Hohlraum 17 des flexiblen Strangs 2 begrenzenden Oberfläche des flexiblen Strangs 2 und der Außenoberfläche des Kupplungsstücks 3 befindet.

Um den Klebstoff 7 in den Verbindungsbereich 9 zu verdrängen, wird ein äußerer Druck auf die den Hohlraum 6 begrenzende Wand 12 des Kupplungsstücks 3 ausgeübt. Die Richtung 11 des ausgeübten Drucks ist in Fig. 6 eingezeichnet. Der unter Druck stehende Klebstoff 7 übt eine entsprechende Kraft auf den Verschluss 10 aus. Bei Erreichen eines Grenzdrucks des Klebstoffs 7 öffnet sich der Verschluss 10 aufgrund der auf diesen einwirkenden Kraft und der Klebstoff 7 kann in den Verbindungsbereich 9 zwischen dem ersten Ende 4 des flexiblen Strangs 2 und dem Kupplungsstück 3 vordringen. Der als Membran ausgebildete Verschluss 10 reißt hierbei durch den auf den Klebstoff 7 einwirkenden und vom Klebstoff 7 übertragenen Druck auf. In anderen Ausführungsbeispielen könnte auch vorgesehen sein, dass die am Kupplungsstück 3 angebrachte, z. B. geklebte Membran zumindest bereichsweise abreißbar ist. Auch könnte vorgesehen sein, dass die Membran ihre Lage aufgrund des Druckes so verändert wird, dass der Klebstoff 7 durch die Klebstoffaustrittsöffnungen 8 austreten kann. Beispielsweise könnte die jeweilige, im Ausführungsbeispiel gemäß Fig. 1 bis Fig. 7 ringförmig ausgebildete Membran eine Elastizität aufweisen und dadurch vom Kupplungsstück 3 abhebbar sein.

Das Kupplungsstück 3 ist im Ausführungsbeispiel gemäß Fig. 1 bis Fig. 7 zum Einbringen des Klebstoffs 7 in den Verbindungsbereich 9 im Bereich des Hohlraumes 6 verformbar, insbesondere elastisch verformbar. Nach dem Einbringen des Klebstoffs 7 nimmt das Kupplungsstück 3 bei Loslassen des Kupplungsstücks 3 also selbstständig wieder im Wesentlichen die Ausgangsform ein. Die Verformung des Kupplungsstücks 3 im Bereich des Hohlraums 6 zum Einbringen des Klebstoffs 7 in den Verbindungsbereich 9 wird vorteilhafterweise mittels entsprechender medizinische Werkzeuge, z.B. einer Zange, welche dem Fachmann an und für sich bekannt sind, vorgenommen. Auch eine direkte händische Betätigung durch den Arzt ist denkbar und möglich. Nach Aushärtung des Klebstoffs 7 ist der dauerhaft verbundene Zustand der Enden 4, 5 der medizinischen Einrichtung 1 unter Ausbildung eines Rings erreicht (Fig. 8).

Vorzugsweise weist die medizinische Einrichtung 1 über die gesamte Längserstreckung des flexiblen Strangs 2, vorzugsweise über ihren gesamten Umfang, eine zumindest im Wesentlichen gleichmäßige Elastizität bzw. Weichheit auf.

Bevorzugterweise weist die medizinische Einrichtung 1 über ihren gesamten Umfang in einem Querschnitt normal zur Längserstreckung des flexiblen Strangs 2 gesehen, eine zumindest im Wesentlichen identische Außenkontur auf. Dadurch kann vorteilhafterweise ein Ring ohne örtliche Verdickung, also mit gleichem Außendurchmesser des Querschnitts des flexiblen Strangs 2 erreicht werden.

Das Kupplungsstück 3 besitzt im Ausführungsbeispiel eine Art Ringbund, welcher sich im verbundenen bzw. implantierten Zustand zwischen dem ersten Ende 4 und dem zweiten Ende 5 des Strangs befindet. Dies ist nicht zwingend, es könnte auch auf einen Bund verzichtet werden, sodass das erste und zweite Ende 4, 5 des flexiblen Strangs direkt aneinander stoßen.

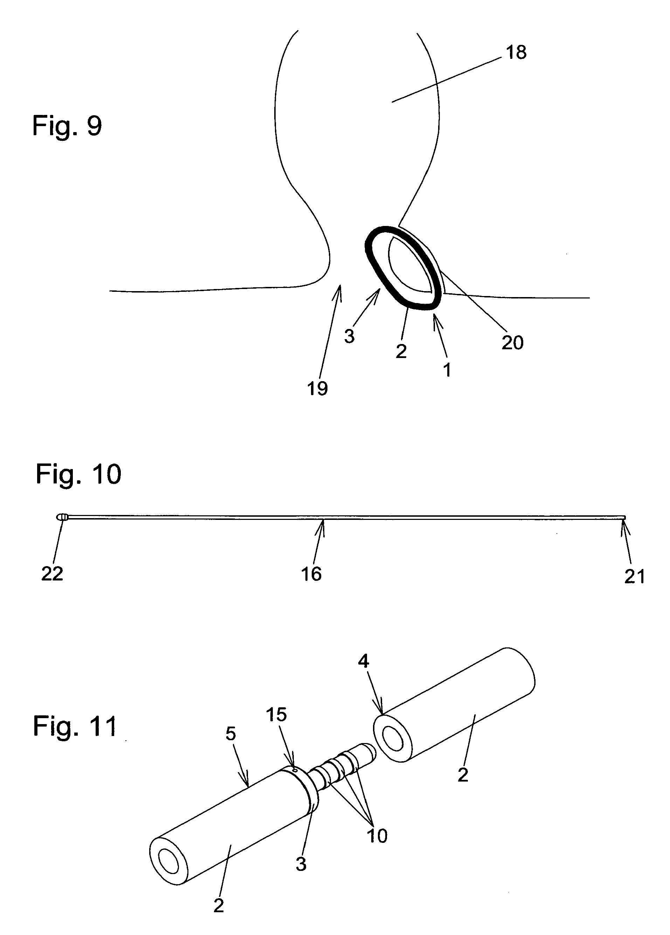

In Fig. 9 ist die Anwendung der medizinischen Einrichtung 1 zur Ausbildung eines implantierbaren Rings zur Drainage von rekto-analen Fisteln, welche sich im Bereich des Schließmuskels des Anus 19 befinden, gezeigt. Bei einer Fistel handelt es sich allgemein um eine nicht natürlich vorbestehende, röhren- oder röhrennetzartige Verbindung zwischen einem inneren Hohlorgan und anderen Organen. Im Fall der dargestellten rekto-analen Fisteln befindet sich ein Fistelgang 20 zwischen dem Rektum 18 und der Haut des Patienten. Fisteln können sich schmerzhaft entzünden. Bevor eine weitere operative Behandlung, z.B. eine Aufspaltung der Fistel, durchgeführt werden kann, ist häufig eine Drainage der Fistel zur Beruhigung der Entzündung nötig.

Mittels einer weiter unten noch beschriebenen Einführsonde 16, vgl. Fig. 10, wird die medizinische Einrichtung vom Arzt in den Fistelgang 20 eingeführt. Der flexible Strang 2 wird beim dargestellten Anwendungsbeispiel vom Rektum 18 über den Anus 19 wieder nach außen geführt und die Einführsonde 16 entfernt. Nach einer optionalen Verkürzung des überstehenden flexiblen Strangs 2 kann der Arzt die Verbindung der beiden Enden 4, 5 des flexiblen Strangs 2 vornehmen. Er führt dazu das erste Ende 4 über das Kupplungsstück 3 und bringt dann beispielsweise mittels einer Zange den erforderlichen Druck zum Öffnen des mindestens einen Verschlusses 10 auf, um den Klebstoff 7 aus dem Hohlraum 6 in den Verbindungsbereich 9 zu verdrängen. Nach der entsprechenden Vorhaltezeit ist die Verbindung der beiden Enden 4, 5 des flexiblen Stranges 2 dauerhaft und zuverlässig hergestellt.

Die in Fig. 10 dargestellte Einführsonde 16 weist ein Ende 21 auf, welches mit dem flexiblen Strang 2 oder dem Kupplungsstück 3 verbindbar ist. Zur Erleichterung der Einführung des flexiblen Strangs 2 in den Fistelgang 20 und um die Verletzungsgefahr für den Patienten gering zu halten, weist die Einführsonde 16 einen abgerundeten Kopf 22 auf. Die Einführsonde 16 könnte bereits im Auslieferungszustand am ersten Ende 4 des flexiblen Strangs 2 oder auch dem Kupplungsstück 3 angebracht sein, insbesondere reibschlüssig, um einen unmittelbaren Einsatz der medizinischen Einrichtung 1 am Patienten zu ermöglichen. Andererseits könnte die Einführsonde 16 aber auch vom medizinischen Personal vor der Implantation der medizinischen Einrichtung 1 mit dem flexiblen Strang 2 oder dem Kupplungsstück 3 verbunden werden. Die Einführsonde 16 besteht vorzugsweise aus einem plastisch verformbaren Material, z.B. Metall.

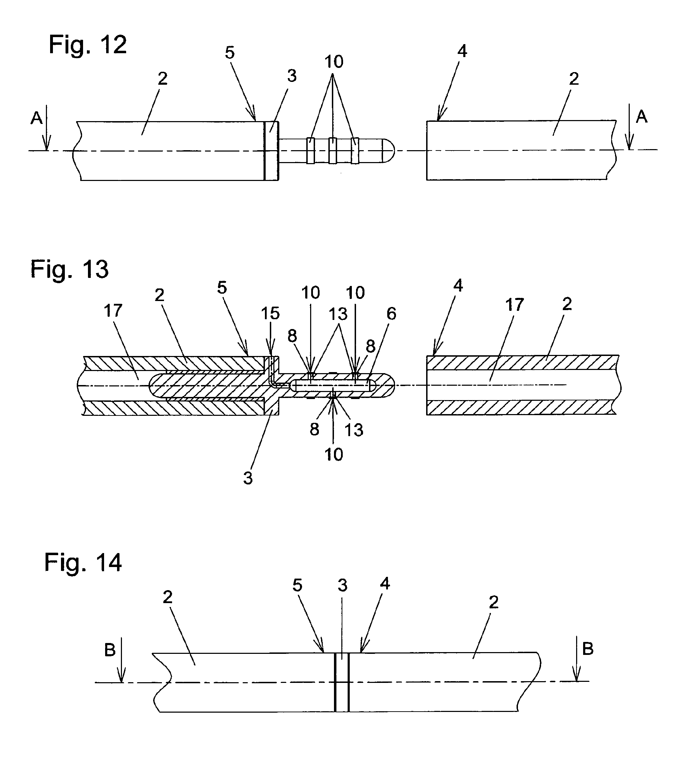

Die Fig. 11 bis 16 zeigen eine zweite Ausgestaltungsform der medizinischen Einrichtung 1. Abgesehen von den im Folgenden beschriebenen Unterschieden treffen die obigen Ausführungen zum ersten, in den Fig. 1 bis 7 dargestellten Ausführungsbeispiel auch auf dieses zweite Ausführungsbeispiel zu.

Im zweiten Ausführungsbeispiel weist das Kupplungsstück 3 im Unterschied zum ersten Ausführungsbeispiel zusätzlich eine Klebstoffeintrittsöffnung 15 auf. Der Hohlraum 6 des Kupplungsstücks 3 ist im Auslieferungszustand der medizinischen Einrichtung 1 ungefüllt. Der Klebstoff 7 wird vom Arzt vor oder während des medizinischen Eingriffs über die Klebstoffeintrittsöffnung 15 dem Hohlraum 6 des Kupplungsstücks 3 und in weiterer Folge der zumindest einen Klebstoffaustrittsöffnung 8 zugeführt. Im Falle von mehreren Klebstoffaustrittsöffnungen 8, wie dies bevorzugt ist, dient der Hohlraum 6 insbesondere der Verteilung des Klebstoffes 7 im Kupplungsstück 3. Auch beim zweiten Ausführungsbeispiel kann eine jeweilige Klebstoffaustrittsöffnung 8 durch einen Verschluss 10 analog zum ersten Ausführungsbeispiel verschlossen sein. Dies ist aber nicht zwingend nötig, vielmehr könnte im zweiten Ausführungsbeispiel auf den Verschluss 10 verzichtet werden. Ein Vorteil des zweiten Ausführungsbeispiels liegt darin, dass die medizinische Einrichtung 1 keinen speziellen Lageranforderungen aufgrund des Klebstoffes 7 unterliegt, da dieser separat gelagert werden kann. Somit ist auch eine über einen längeren Zeitraum sich erstreckende Lagerung der medizinischen Einrichtung 1 möglich. Der Arzt kann die jeweils benötigte Menge des Klebstoffes 7 durch Ansetzen einer Kanüle einer medizinischen Spritze an der Klebstoffeintrittsöffnung 15 in das Kupplungsstück 3 injizieren bis der Klebstoff 7 an den Klebstoffaustrittsöffnungen 8 austritt und so die dauerhafte und zuverlässige Verbindung der beiden Enden 4, 5 des flexiblen Strangs 2 herstellen.

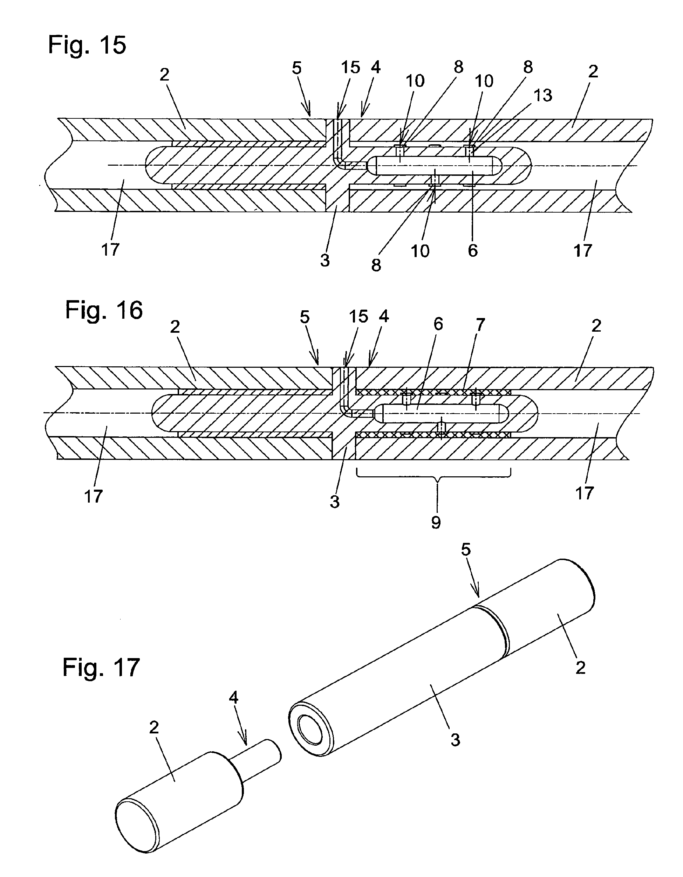

Eine weitere Ausführungsform ist in den Fig. 17 bis 22 dargestellt. Abgesehen von den im Folgenden beschriebenen Unterschieden treffen die obigen Ausführungen zum ersten, in den Fig. 1 bis 7 dargestellten Ausführungsbeispiel auch auf dieses Ausführungsbeispiel zu.

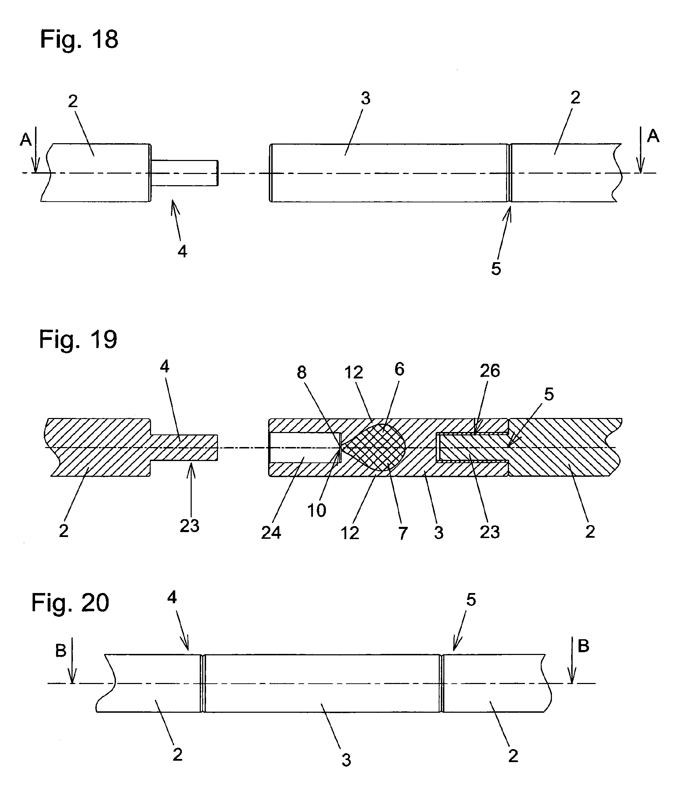

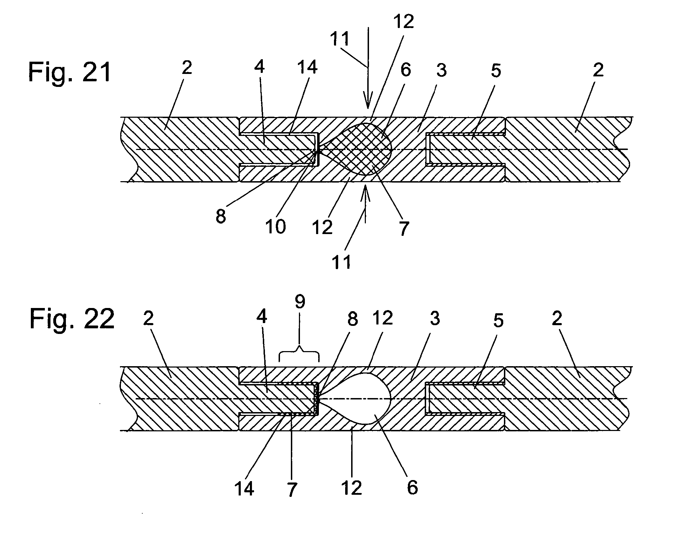

Der flexible Strang 2 besteht in dieser Ausführungsform zumindest im Bereich der Enden 4, 5 aus Vollmaterial. Das erste und zweite Ende 4, 5 des flexiblen Strangs 2 weisen jeweils eine Fortsatz 23 auf, vgl. Fig. 19. Der Fortsatz 23 ragt im verbundenen Zustand der beiden Enden 4, 5 jeweils in einen Hohlraum 24, 26 des Kupplungsstücks 3, vgl. Fig. 21 und Fig. 22. Das Kupplungsstück 3 weist analog zum ersten Ausführungsbeispiel einen Hohlraum 6 auf, welcher bereits im Auslieferungszustand mit Klebstoff 7 aufgefüllt ist. Die Klebstoffaustrittsöffnung 8 befindet sich auf einer Stirnseite des Hohlraumes 24 des Kupplungsstücks 3 und ist mit einem Verschluss 10 in Form einer Membran verschlossen. Nach der Ausbildung des Rings durch Einstecken des Fortsatzes 23 des ersten Endes 4 in den Hohlraum 24 des Kupplungsstücks 3 kann durch Aufbringen des erforderlichen Drucks in Richtung 11 der Klebstoff 7 nach zumindest bereichsweiser Ablösung des Verschlusses 10 vom Kupplungsstück 3 oder dem Aufreißen des Verschlusses 10 dem Verbindungsbereich 9 zwischen dem ersten Ende 4 des flexiblen Strangs 2 und dem Kupplungsstück 3 zugeführt werden. Der Hohlraum 6 schließt in diesem Ausführungsbeispiel im Wesentlichen direkt an die Austrittsöffnung 8 an, d. h. dass bei dieser Ausgestaltung auf einen ausgeprägten Gang zwischen dem Hohlraum 6 und der Klebstoffaustrittsöffnung 8 verzichtet wurde. Es könnte aber auch hier ein entsprechender Gang vorgesehen sein, wie auch bei der ersten und zweiten Ausgestaltungsform auf eben diesen verzichtet werden könnte.

Der flexible Strang 2 wäre in diesem Ausführungsbeispiel günstigerweise bereits in seiner Länge vorkonfektioniert, um die Herstellung des Fortsatzes 23 des ersten Endes 4 während der Implantation zu vermeiden. Der mit Klebstoff 7 aufzufüllende Spalt 14 ist bei diesem Ausführungsbeispiel im verbundenen Zustand des ersten und zweiten Endes 4, 5 der medizinischen Einrichtung 1 sowohl zwischen einer Mantelfläche als auch der Stirnfläche des flexiblen Strangs 2 und einer den Hohlraum 24 begrenzenden Oberfläche des Kupplungsstücks 3 angeordnet.

Der flexible Strang 2 besteht aus einem langzeitimplantatfähigen Material, d.h., die medizinische Einrichtung 1 ist geeignet, für einen Zeitraum der länger als 30 Tage ist, im oder am Körper des Patienten angewendet zu werden. Solche Materialien sind dem Fachmann hinlänglich bekannt, beispielsweise könnte es sich um Polyurethan, Nylon, Polypropylen, Polytetrafluorethylen oder Silikon handeln.

Der Außendurchmesser des flexiblen Strangs 2 ist an die konkrete Anwendung angepasst und kann z.B. bei der Anwendung zur Drainage günstigerweise im Bereich von 0,5 mm bis 2 mm liegen. In anderen Anwendungen kann der Außendurchmesser im Bereich von 0,5mm bis 30mm liegen. In den Ausführungsbeispielen ist vorgesehen, dass der Querschnitt des flexiblen Strangs 2 kreisförmig ist. In anderen Ausgestaltungen der Erfindung könnte vorgesehen sein, dass der Querschnitt von der Kreisform abweicht und beispielsweise eine elliptische oder rechteckige Form aufweist.

Das Kupplungsstück 3 besteht vorzugsweise aus Kunststoff, beispielsweise aus einem der zuvor im Zusammenhang mit dem flexiblen Strang 2 genannten. Die Membran des Verschlusses 10 wird in den in den Figuren dargestellten Ausführungsbeispielen von einer Folie gebildet, die z. B. aus Kunststoff oder auch Metall bestehen kann.

Beim Klebstoff 7 handelt es sich insbesondere um einen handelsüblichen Klebstoff, der geeignet ist, das Kupplungsstück 3 mit den Enden 4, 5 des flexiblen Strangs 2 zu verbinden. Der Verklebevorgang kann bei entsprechenden Klebstoffen durch Einbringen von Wärme beschleunigt werden.

Es ist denkbar und möglich, dass die Verbindung zwischen dem Kupplungsstück 3 und dem ersten Ende 4 des flexiblen Strangs 2 neben einer reinen Klebeverbindung auch eine Kombination einer Klebeverbindung mit einer reibschlüssigen und/oder formschlüssigen Verbindung ist. Dadurch kann bereits vor dem Einbringen des Klebstoffs 7 in den Verbindungsbereich 9 die relative Lage des ersten Endes 4 des flexiblen Strangs 2 und des Kupplungsstücks 3 zueinander definiert und gehalten werden, ohne dass sich die Verbindung selbsttätig lösen kann.

Es ist es auch denkbar und möglich, dass das Kupplungsstück 3 Klebstoffaustrittsbohrungen zur Zuführung von Klebstoff 7 in einen Verbindungsbereich zwischen dem zweiten Ende 5 des flexiblen Strangs 2 und dem Kupplungsstück 3 aufweist.

Neben der Anwendung der medizinischen Einrichtung 1 zum Drainieren von rekto-analen Fisteln kann ein in erfindungsgemäßer Weise implantierbarer Ring z. B. auch zum Einschnüren von Hohlorganen oder Markieren von Gewebe verwendet werden. Auch in diesen Fällen wird eine Klebeverbindung zwischen dem ersten Ende 4 des flexiblen Strangs 2 und dem Kupplungsstück 3 hergestellt.

Zur Ausbildung eines implantierbaren Rings zum Markieren von Gewebe kann der flexible Strang 2 auch ein geeignetes Markermaterial aufweisen.

### Legende zu den Hinweisziffern:

- 1: medizinische Einrichtung
- 2: flexibler Strang
- 3: Kupplungsstück
- 4: erstes Ende
- 5: zweites Ende
- 6: Hohlraum
- 7: Klebstoff
- 8: Klebstoffaustrittsöffnung
- 9: Verbindungsbereich
- 10: Verschluss
- 11: Richtung
- 12: Wand
- 13: Gang
- 14: Spalt
- 15: Klebstoffeintrittsöffnung
- 16: Einführsonde
- 17: Hohlraum
- 18: Rektum
- 19: Anus
- 20: Fistelgang
- 21: Ende
- 22: Kopf
- 23: Fortsatz
- 24: Hohlraum
- 25: Klebstoff
- 26: Hohlraum

## Patentansprüche

1. Medizinische Einrichtung (1) zur Ausbildung eines implantierbaren Rings, insbesondere zur Drainage oder zum Einschnüren oder Markieren von Gewebe, umfassend einen flexiblen Strang (2) und zumindest ein Kupplungsstück (3) zum Verbinden eines ersten Endes (4) und eines zweiten Endes (5) des flexiblen Strangs (2), **dadurch gekennzeichnet, dass** das Kupplungsstück (3) einen Hohlraum (6) zur Aufnahme von Klebstoff (7) aufweist und vom Hohlraum (6) durch zumindest eine Klebstoffaustrittsöffnung (8) Klebstoff (7) in einen Verbindungsbereich (9) zwischen dem ersten Ende (4) des flexiblen Strangs (2) und dem Kupplungsstück (3) zuführbar ist.

2. Medizinische Einrichtung (1) nach Anspruch 1, **dadurch gekennzeichnet, dass** das zweite Ende (5) des flexiblen Strangs (2) mit dem Kupplungsstück (3) reibschlüssig und/oder formschlüssig und/oder stoffschlüssig verbunden ist.

3. Medizinische Einrichtung (1) nach Anspruch 1 oder 2, **dadurch gekennzeichnet, dass** der Hohlraum (6) des Kupplungsstücks (3) mit Klebstoff (7) aufgefüllt ist und zumindest ein Verschluss (10) zum Verhindern eines ungewollten Austritts von Klebstoff (7) durch die zumindest eine Klebstoffaustrittsöffnung (8) vorgesehen ist.

4. Medizinische Einrichtung (1) nach Anspruch 3, **dadurch gekennzeichnet, dass** zum Ausdrücken von Klebstoff (7) durch die zumindest eine Klebstoffaustrittsöffnung (8) zumindest eine den Hohlraum (6) begrenzende Wand (12) durch einen auf das Kupplungsstück (3) ausgeübten äußeren Druck in den Hohlraum (6) eindrückbar ist.

5. Medizinische Einrichtung (1) nach Anspruch 4, **dadurch gekennzeichnet, dass** der Verschluss (10) durch einen auf den Klebstoff (7) einwirkenden und vom Klebstoff (7) auf den Verschluss (10) übertragenen Druck öffenbar ist.

6. Medizinische Einrichtung (1) nach einem der Ansprüche 3 bis 5, **dadurch gekennzeichnet, dass** der Verschluss (10) von einer Membran gebildet wird und die Membran durch einen auf den Klebstoff (7) einwirkenden und vom Klebstoff (7) übertragenen Druck aufreißbar oder vom Kupplungsstück (3) zumindest bereichsweise abreißbar oder abhebbar ist.

7. Medizinische Einrichtung (1) nach Anspruch 1 oder 2, **dadurch gekennzeichnet, dass** das Kupplungsstück (3) zumindest eine Klebstoffeintrittsöffnung (15) zum Einbringen von Klebstoff (7) in den Hohlraum (6) des Kupplungsstücks (3) aufweist.

8. Medizinische Einrichtung (1) nach einem der Ansprüche 1 bis 7, **dadurch gekennzeichnet, dass** das Kupplungsstück (3) zumindest im Bereich des Hohlraumes (6) zum Einbringen des Klebstoffs (7) in den Verbindungsbereich (9) elastisch verformbar ist.

9. Medizinische Einrichtung (1) nach einem der Ansprüche 1 bis 8, **dadurch gekennzeichnet, dass** im zusammengesteckten Zustand des Kupplungsstücks (3) und des ersten Endes (4) des flexiblen Strangs (2) zwischen einer einen Hohlraum (17) des flexiblen Strangs (2) begrenzenden Oberfläche des flexiblen Strangs (2) und einer Außenoberfläche des Kupplungsstücks (3) ein mit Klebstoff (7) befüllbarer Spalt (14) vorhanden ist.

10. Medizinische Einrichtung (1) nach einem der Ansprüche 1 bis 9, **dadurch gekennzeichnet, dass** der flexible Strang (2) in Form eines Schlauchs mit einem in Längsrichtung durchgehenden Hohlraum (17) ausgebildet ist.
